(19) Europäisches Patentamt
European Patent Office
Office européen des brevets

(11) EP 1 593 732 A1

(12) **DEMANDE DE BREVET EUROPEEN**

(43) Date de publication:
**09.11.2005 Bulletin 2005/45**

(51) Int Cl.⁷: **C11C 3/10**, C07C 67/03, C07C 69/24, C07C 69/52

(21) Numéro de dépôt: **05290798.7**

(22) Date de dépôt: **11.04.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **03.05.2004 FR 0404730**
**03.05.2004 FR 0404731**

(71) Demandeur: **Institut Français du Pétrole**
**92852 Rueil Malmaison Cédex (FR)**

(72) Inventeurs:
- **Delfort, Bruno**
**75005 Paris (FR)**
- **Hillion, Gerard**
**95220 Herblay (FR)**
- **Le Pennec, Dominique**
**78910 Orgerus (FR)**
- **Lendresse, Christophe**
**92500 Rueil Malmaison (FR)**

(54) **Procede de transesterification d'huiles vegezales ou animales au moyen de catalyseurs heterogenes a base de zinc ou de bismuth de titane et d'aluminium**

(57) Un nouveau procédé de fabrication d'esters d'acides monocarboxyliques linéaires de 6 à 26 atomes de carbone, par réaction d'huiles végétales ou animales, neutres ou non, avec des monoalcools de 1 à 18 atomes de carbone, utilise un catalyseur choisi parmi :

- les combinaisons d'oxydes de zinc et de titane ;
- les combinaisons d'oxyde de zinc, d'oxyde de titane et d'alumine ;
- les combinaisons d'oxydes de bismuth et de titane ; et
- les combinaisons d'oxyde de bismuth, d'oxyde de titane et d'alumine.

Le procédé permet de fabriquer directement, en une ou plusieurs étapes, un ester utilisable comme carburant ou combustible et une glycérine pure.

EP 1 593 732 A1

**Description**

La présente invention est relative à un nouveau procédé de fabrication d'esters d'acides monocarboxyliques à partir d'huiles végétales ou animales.

La réaction qui est visée de façon principale est une transestérification réalisée selon le schéma 1 ci-dessous et éventuellement une réaction couplée estérification et transestérification, l'estérification étant réalisée selon le schéma II ci-dessous. Dans ces schémas, les chaînes d'acides gras sont arbitrairement représentées par des chaînes de type oléique.

- Schéma I

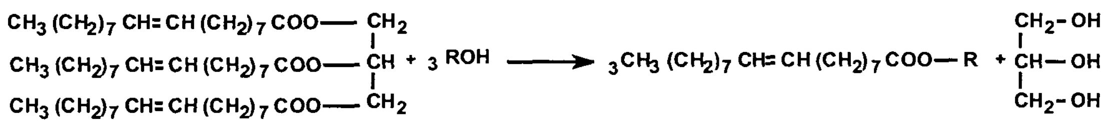

- Schéma II

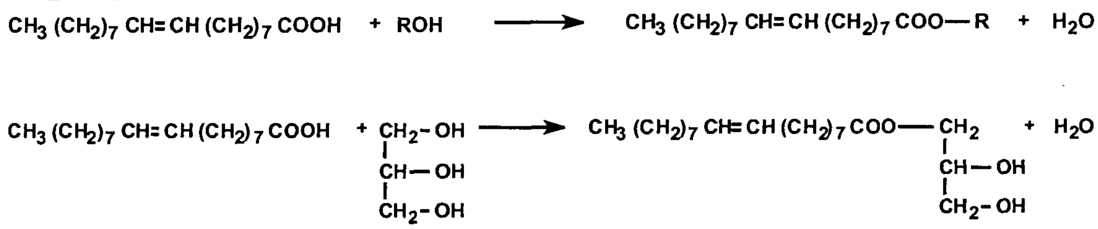

Les esters de corps gras sont actuellement utilisés dans de nombreuses applications comme carburants diesel, fuels domestiques, solvants, composés de base pour la fabrication de sulfonates d'alcools gras, d'amides, de dimères d'esters, etc.

Lorsqu'on fabrique un ester à partir d'huile et de monoalcool, il se forme automatiquement, selon la nature de l'huile engagée au départ, de 10 à 15 % d'un produit secondaire, qui est la glycérine. Cette glycérine est vendue à un prix élevé pour des utilisations variées, mais seulement lorsqu'elle a une grande pureté. Celle-ci est obtenue après des purifications poussées dans des unités spécialisées dans la distillation sous vide.

Dans la fabrication d'esters méthyliques de corps gras à partir d'huiles raffinées et d'alcool, alors qu'on utilise couramment comme catalyseurs des dérivés alcalins simples, comme les alcoolates de sodium, la soude ou la potasse, dans des conditions assez douces (température de 50 à 80 °C et pression atmosphérique), ainsi qu'on peut le lire dans de nombreux brevets ou publications, par exemple dans le JAOCS 61, 343-348 (1984), on n'arrive cependant à un produit pur utilisable comme carburant et une glycérine aux normes qu'après de très nombreuses étapes.

Si l'on prend par exemple les catalyseurs alcalins les plus utilisés, on retrouve ces composés alcalins aussi bien dans la glycérine que dans l'ester. Il faut les éliminer par lavage et/ou par neutralisation dans la fraction ester, puis sécher celle-ci. Dans la phase glycérine, il faut neutraliser les savons et les alcoolates présents, parfois éliminer les sels formés.

La glycérine ainsi obtenue contient de l'eau, généralement entre 5 % et 40 % en masse. Elle contient également les sels issus de la neutralisation du catalyseur alcalin, par exemple du chlorure de sodium lorsque le catalyseur est la soude ou le méthylate de sodium et lorsque la neutralisation est effectuée avec de l'acide chlorhydrique. La concentration en sels dans la glycérine issue de ces procédés est généralement de 3 % à 6 % en masse. L'obtention de glycérol de grande pureté à partir de glycérine issue de ces procédés impose donc des étapes de purification comme la distillation sous pression réduite, que l'on peut parfois associer à des traitements sur résines échangeuses d'ions.

En résumé, la plupart des procédés commerciaux de fabrication d'esters aboutissent assez facilement à des produits bruts (esters et glycérine), qu'il faut cependant purifier de façon approfondie par divers traitements qui grèvent finalement le prix de la transformation.

On a maintenant découvert, de façon surprenante, que l'on pouvait obtenir en 1 à 3 étapes, dans des conditions particulières, directement à partir d'huiles végétales ou animales et de monoalcools, des esters de ces monoalcools et une glycérine exempte de sels, en tout cas n'en contenant pas plus de 5 ppm, d'une pureté de 95 % à 99,9 % le plus souvent de 98 % à 99,9 %, et ce en utilisant comme catalyseurs, soit en continu, par exemple en lit fixe, soit en discontinu, un système catalytique hétérogène particulier.

L'utilisation de catalyseurs hétérogènes n'est pas nouvelle.

Parmi les documents antérieurs qui traitent de catalyseurs hétérogènes, on peut citer le brevet européen EP-B-0 198 243. Le catalyseur de transestérification, qui transforme huile et méthanol en ester méthylique, est une alumine ou un mélange d'alumine et d'oxyde ferreux. Dans les exemples, la colonne utilisée pour le lit fixe a un volume de 10 litres et l'on injecte généralement de l'huile à un débit inférieur à 1 litre/heure, ce qui donne une WH (WH = volume d'huile injecté/ volume de catalyseur/ heure) inférieure à 0,1. Pour une usine de 100 000 t/an, cela correspondrait à des réacteurs d'au moins 150 $m^3$.

Un autre problème qui semble se poser est celui de la quantité de glycérine recueillie, très inférieure à la théorie. La température de réaction élevée (280°C à 320°C) cause une dégradation du glycérol. Des exemples indiquent des teneurs en éthers de glycérol supérieurs à 80% par rapport au glycérol formé. Dans aucun exemple où l'on est censé recueillir 10 % de glycérine, on n'obtient, même de manière approchée, cette valeur. Enfin, la pureté des esters est assez faible, de 93,5 à 98 %. Dans certains cas, il se forme des quantités importantes d'éthers de glycérine, comme cela est signalé dans ce brevet ; dans d'autres cas, peut-être se décompose t'elle, à moins qu'elle ne soit éliminée dans une première étape. Le niveau de performance est donc assez bas. On peut signaler qu'aux WH indiquées et pour des temps de contact de plus de 6 heures, on peut obtenir même sans catalyseur des conversions de 80 % et plus.

Ce brevet ne semble donc pas présenter une solution raisonnable du point de vue économique.

Il existe d'autres références dans la littérature qui mentionnent cette fois-ci l'oxyde de zinc, toutefois dans des réactions d'estérification de la glycérine avec un acide gras [Osman in "*Fette Seifen und Anstrichmittel"* 331-33 (1968)]. Dans ce travail, on compare une vingtaine de catalyseurs à 180 °C dans un procédé en discontinu. Il n'y a pratiquement aucune différence entre le chlorure de zinc, le sulfate de zinc, la poudre de zinc, l'oxyde de baryum, de calcium, l'oxyde de zinc, l'alumine, l'acide thiosalicylique, le phosphate de calcium, le bicarbonate de potassium, le méthylate ou l'éthylate de sodium et même l'hydroxyde de lithium. Tous ces sels ou oxydes donnent entre 32 et 39 % de rendement en monoglycéride dans un essai comparatif où l'on utilise un excès de glycérine par rapport à l'acide gras.

On peut citer le brevet US-A-5 908 946 qui décrit un procédé qui peut fonctionner en continu ou en discontinu et utilisant des catalyseurs solides et non solubles. Toutefois, les catalyseurs utilisés sont soit de l'oxyde de zinc, soit un mélange d'oxyde de zinc et d'alumine, soit un aluminate de zinc.

La présente invention propose un procédé de fabrication d'au moins un ester d'acide gras et de glycérine, ces deux produits étant obtenus à un haut degré de pureté, ce procédé pouvant être défini d'une manière générale par le fait que l'on opère, par réaction d'huiles végétales ou animales, acides ou neutres, avec des monoalcools, par exemple de 1 à 18 atomes de carbone, de préférence de 1 à 12 atomes de carbone, et de manière encore plus préférée de 1 à 5 atomes de carbone, en utilisant au moins un catalyseur choisi parmi :

- les combinaisons d'oxydes de zinc et de titane ;
- les combinaisons d'oxyde de zinc, d'oxyde de titane et d'alumine ;
- les combinaisons d'oxydes de bismuth et de titane ; et
- les combinaisons d'oxyde de bismuth, d'oxyde de titane et d'alumine.

Les combinaisons d'oxydes comprenant de l'oxyde de zinc, de l'oxyde de titane et éventuellement de l'alumine répondent plus particulièrement à la formule :

$$[(ZnO)_a - (TiO_2)_b]y\ [Al_2O_3]1\text{-}y$$

cette formule pouvant également prendre la forme :

$$[Zn_aTi_bO_{(a+2b)}]y\ [Al_2O_3]1\text{-}y$$

(a ayant une valeur de 0,5 à 5, b ayant une valeur de 0,5 à 5 et y ayant une valeur de 0,005 à 1, de préférence de 0,005 à 0,995).

Les combinaisons d'oxydes comprenant de l'oxyde de bismuth, de l'oxyde de titane et éventuellement de l'alumine répondent plus particulièrement à la formule :

$$[(Bi_2O_3)_a - (TiO_2)_b]y\ [Al_2O_3]1\text{-}y$$

(a ayant une valeur de 0,5 à 5, b ayant une valeur de 0,5 à 5 et y ayant une valeur de 0,005 à 1, de préférence de 0,005 à 0,995)

Les conditions de la réaction des huiles végétales ou animales avec les monoalcools incluent préférentiellement une température de 150 à 250 °C et une pression inférieure à 100 bar et de préférence de 10 à 70 bar (on rappelle que 1 bar = 0,1 MPa), en utilisant un excès de monoalcool par rapport à la stoechiométrie huile/alcool.

Tous les catalyseurs considérés sont sous forme de poudre, de billes, d'extrudés ou de pastilles. L'utilisation de l'alumine a deux effets favorables. Le premier est d'augmenter la surface spécifique du catalyseur car l'oxyde de zinc (ou de bismuth), le dioxyde de titane ou le titanate de zinc (ou de bismuth) sont connus pour posséder de faibles surfaces spécifiques. Le second est de créer un composé beaucoup plus stable, surtout vis-à-vis de conditions dans lesquelles le composé de titane et le composé de zinc (ou de bismuth) auraient tendance à former des savons de titane ou de zinc (ou de bismuth).

Un intérêt majeur de ces catalyseurs solides est qu'ils catalysent les réactions de transestérification et d'estérification selon un processus de catalyse hétérogène, c'est à dire que le catalyseur solide utilisé d'une part n'est pas consommé dans la réaction et d'autre part n'est jamais dissous dans le milieu réactionnel mais reste sous la forme solide et sera donc séparé du milieu réactionnel liquide sans perte de catalyseur et sans pollution du milieu réactionnel par la présence de catalyseur ou de résidu de catalyseur.

Ceci est vérifié dans l'invention par l'absence de traces provenant du catalyseur aussi bien dans l'ester formé que dans la glycérine produite.

La charge de ce catalyseur n'est pas affectée par la réaction de transestérification ou d'estérification. Son activité catalytique est conservée après la réaction. Ce type de catalyseur est compatible avec une utilisation dans un procédé industriel en continu par exemple en lit fixe dans lequel la charge de catalyseur peut être utilisée pendant une très longue durée sans perte d'activité.

L'ester et le glycérol obtenus ne contiennent pas d'impuretés issues du catalyseur. De ce fait, aucun traitement de purification ne sera à appliquer pour éliminer le catalyseur ou les résidus de celui-ci, contrairement au procédés utilisant des catalyseurs fonctionnant selon un processus homogène, où le catalyseur ou ses résidus sont, après réaction, localisés dans la même phase que l'ester et/ou que la glycérine.

Par la mise en oeuvre de ce procédé, l'épuration finale est réduite au minimum, tout en permettant d'obtenir un ester qui est aux spécifications carburant, et une glycérine de pureté de 95 % à 99,9 % et de préférence entre 98 % et 99,9 %.

Le procédé de l'invention est décrit de manière plus détaillée ci-après.

Parmi les huiles utilisables dans le procédé de l'invention, on peut citer toutes les huiles courantes, comme les huiles de palme, de palmiste, de coprah, de babassu, de colza ancien ou nouveau, de tournesol, de maïs, de ricin et de coton, les huiles d'arachide, de lin et de crambe et toutes les huiles issues par exemple du tournesol ou du colza par modification génétique ou hybridation.

On peut même utiliser des huiles de friture, des huiles animales variées, comme les huiles de poisson, le suif, le saindoux, d'équarrissage et même des graisses.

Parmi les huiles utilisées, on peut encore indiquer des huiles partiellement modifiées par exemple par polymérisation ou oligomérisation comme par exemple les "standolies" d'huiles de lin, de tournesol et les huiles végétales soufflées.

La présence d'acide gras dans les huiles n'est pas a priori préjudiciable, sinon qu'il y a risque de saponification. Il est possible de faire précéder la réaction de transestérification d'une réaction d'estérification, de préférence avec de la glycérine, pour former, à pression atmosphérique ou sous un vide partiel et à des températures de 180 à 220 °C, un glycéride total ou partiel à partir des acides gras.

La nature de l'alcool mis en jeu dans le procédé de l'invention joue un rôle important dans l'activité de la transestérification. D'une manière générale, on peut mettre en jeu divers monoalcools aliphatiques renfermant par exemple de 1 à 18 atomes de carbone, de préférence de 1 à 12 atomes de carbone. Le plus actif est l'alcool méthylique. Toutefois, l'alcool éthylique et les alcools isopropylique, propylique, butylique, isobutylique et même amylique, peuvent être engagés. On peut également utiliser des alcools plus lourds comme l'alcool éthyl-hexylique ou l'alcool laurique. On peut avec avantage ajouter aux alcools lourds de l'alcool méthylique, qui semble faciliter la réaction. Par ailleurs, lorsqu'on prépare l'ester éthylique, on peut mettre en jeu de 1 à 50 %, de préférence de 1 à 10 %, d'alcool méthylique de manière à augmenter la conversion.

La préparation de catalyseurs à base de titane n'est pas nouvelle. On peut citer le brevet US-A-4 490 479, qui traite de la synthèse de catalyseur par co-malaxage. En particulier, les auteurs décrivent l'ajout d'oxydes, d'hydroxydes, d'alcoxydes ou de sels de titane à un précurseur d'alumine ou à des composés d'aluminium hydratés. En présence d'eau et d'acides minéraux ou organiques, le mélange des éléments précités s'effectue afin de former une

pâte. Celle-ci est mise en forme afin d'obtenir un support. Il est également décrit, dans une seconde étape, l'ajout d'un composé du molybdène.

Le brevet US-A-5 169 822 enseigne la déposition en milieu non aqueux d'alcoxydes de titane sur des supports inorganiques (entre autres).

L'article de S. Kumar et al. *in* Mat. Lett. 43 (2000) 286 enseigne la précipitation d'un sol de boehmite avec un sol de dioxyde de titane. Le sol de titane est préparé par stabilisation à l'acide acétique.

Pour produire un catalyseur de formule brute

$$[(ZnO)_a - (TiO_2)_b]y\ [Al_2O_3]1\text{-}y$$

cette formule pouvant également prendre la forme

$$[Zn_aTi_bO_{(a+2b)}]y\ [Al_2O_3]1\text{-}y$$

(a, b et y étant définis comme ci-dessus), on peut utiliser les sources suivantes.

On peut citer parmi les sources de zinc les formes oxydes (ZnO), alcoxydes [$Zn(OR)_2$, avec R = Me, Et, Pr, iPr, Bu, iBu, etc.]. Il est également possible d'utiliser le zinc sous forme de sels inorganiques ($ZnCl_2$, etc.). De même, les formes colloïdales du zinc peuvent être utilisées (par colloïdale, le demandeur entend que la taille des particules d'oxyde de bismuth est de 1 nm à 100 nm). Enfin, les sources de zinc peuvent être des gels issus de l'hydrolyse des sources précédentes, obtenant ainsi une forme d'oxyde de zinc hydratée de formule chimique $Zn(OH)_2$.

Pour produire un catalyseur de formule brute :

$$[(Bi_2O_3)_a - (TiO_2)_b]y\ [Al_2O_3]1\text{-}y$$

(a, b et y étant définis comme ci-dessus), on peut utiliser les sources suivantes.

On peut citer parmi les sources de bismuth les formes oxydes (Bi2O3), alcoxydes [$Bi(OR)_3$, avec R = Me, Et, Pr, iPr, Bu, iBu, etc.]. Il est également possible d'utiliser le bismuth sous forme de sels inorganiques ($BiCl_3$, BiOCl, etc.). De même, les formes colloïdales du bismuth peuvent être utilisées. Enfin, les sources de bismuth peuvent être des gels issus de l'hydrolyse des sources précédentes, obtenant ainsi un hydroxyde de bismuth $Bi(OH)_3$ ou une forme d'oxyde de bismuth partiellement hydratée de formule chimique $Bi_2O_3$,zH2O, avec z compris entre 0 et 10. Il est également avantageux d'utiliser de l'oxyde de bismuth déshydraté, amorphe ou cristallisé.

On peut citer parmi les sources de titane les formes oxydes ($TiO_2$), alcoxydes [$Ti(OR)_4$ avec R = Me, Et, Pr, iPr, Bu, iBu, etc.]. Il est également possible d'utiliser le titane sous forme de sels inorganiques ($TiCl_4$, $TiOSO_4$, $TiOCl_2$, etc.). De même, les formes colloïdales du titane peuvent être utilisées (par colloïdale, le demandeur entend que la taille des particules d'oxyde ou oxyhydroxyde de titane soit compris entre 1 nm et 100 nm). Enfin, les sources de titane peuvent être des gels issus de l'hydrolyse des sources précédentes, obtenant ainsi une forme d'oxyde de titane partiellement hydratée de formule chimique $TiO_2$,zH2O, avec z compris entre 0 et 5. Il est également avantageux d'utiliser de l'oxyde de titane déshydraté, amorphe ou cristallisé, qui possède dans ce dernier cas des structures cristallographiques quadratique, monoclinique ou cubique connues par l'homme du métier.

<u>Sources d'alumine</u>

Les sources d'aluminium utilisées dans l'invention peuvent être de forme alcoxyde de formule générale $Al(OR)_3$, avec R = Me, Et, Pr, iPr, Bu, iBu, etc. ou hydoxyde. Les sels inorganiques d'aluminium peuvent également être avantageusement utilisés, à savoir les chlorures, nitrates, sulfates, etc. De même, la source d'aluminium peut être basique, dans lequel cas l'aluminium est sous forme aluminate ($AlO_2^-$). Le contre-ion peut être un alcalin (Li, Na, K, Cs) et plus généralement tout contre-ion positif (NH4+ par exemple). Dans le cas de l'utilisation d'un précurseur solide d'aluminium, tout composé d'alumine de formule générale $Al_2O_3$, $nH_2O$ peut être utilisé. Sa surface spécifique est de 100 à 600 $m^2/g$. On peut en particulier utiliser des composés hydratés de l'alumine tels que l'hydrargillite, la gibbsite, la bayerite, la boehmite, la pseudo-boehmite et les gels d'alumines amorphes ou essentiellement amorphes. On peut également mettre en oeuvre les formes déshydratées de ces composés qui sont constitués d'alumines de transition et qui comportent au moins une phase prise dans le groupe : rhô, khi, êta, kappa, thêta, delta, gamma et alpha, qui se différencient essentiellement sur l'organisation de leur structure cristalline.

Le catalyseur peut être avantageusement préparé par l'une des méthodes décrites ci-après.

1. L'imprégnation d'au moins un sel soluble, d'un alcoxyde, d'un sol ou d'un alcoxyde sur un support d'alumine préformé de surface spécifique de 20 à 600 $m^2/g$, de préférence entre 100 et 370 $m^2/g$. Ce support peut être sous forme de poudre, de billes, d'extrudés ou tout autre forme connue de l'homme du métier et permettant de travailler en lit fixe, en lit bouillonnant ou en slurry. Ce support est choisi parmi les sources d'alumines précitées. Après diverses étapes connues de l'homme du métier, les catalyseurs sont séchés entre 25 °C et 150 °C, de manière préférée entre 50 °C et 120 °C, puis calcinés à des températures comprises entre 150 et 1000 °C, de manière préférée entre 250 et 600 °C.

2. Le malaxage d'au moins un composé de titane et d'au moins un composé du zinc (ou de bismuth) avec un composé d'alumine plus ou moins hydratée définis au dessus en tant que précurseur solide en présence d'un agent peptisant (acide minéral ou acide organique). De manière préférée, les agents peptisants sont les acides nitrique et acétique. Il peut également être ajouté à la pâte obtenue des agents connus pour faciliter la mise en forme tel que les dérivés de type méthyl cellulose ou tous autres composés connus de l'homme du métier pour cet effet. Le produit est ensuite mis en forme par extrusion, puis séché entre 40 et 150 °C, de manière préférée entre 70 et 120 °C, puis calciné à des températures comprises entre 300 et 1100 °C, de manière préférée entre 350 et 800 °C.

3. La synthèse par voie sol-gel entre un alcoxyde de zinc (ou de bismuth), un alcoxyde de titane et un alcoxyde d'aluminium, choisis parmi les sources citées plus haut, de préférence, pour l'aluminium, le sec-butoxyde ; pour le zinc, l'isopropoxyde ; pour le bismuth, le pentyloxyde ; et, pour le titane, l'isopropoxyde. Ces précurseurs peuvent être mélangés en présence d'un solvant approprié et éventuellement d'agent complexant ou de surfactants. Le tout peut être hydrolysé afin d'obtenir un gel. Le gel peut être séché entre 40 et 140 °C, de préférence entre 80 et 130 °C et mis en forme par les techniques classiques d'extrusion avec ajout éventuel de liant, soit par remise en suspension dans un liquide adéquat pour former des billes par précipitation « oil-drop », soit pastillé. Dans tous les cas, les objets mis en forme sont séchés entre 40 et 150 °C, de manière préférée entre 70 et 120 °C, puis calcinés à des températures comprises entre 300 et 1100 °C, de manière préférée entre 350 et 800 °C.

4. La co-précipitation entre au moins un sel de zinc (ou de bismuth), un sol ou un alcoxyde de zinc (ou de bismuth), au moins un sel de titane, un sol ou un alcoxyde de titane et au moins un sel, un sol ou un alcoxyde d'aluminium en voie aqueuse. La co-précipitation peut avoir lieu en présence uniquement d'eau ou d'agents favorisant la précipitation tels une base inorganique (soude, potasse, carbonate de sodium, ammoniaque, hydrazine, etc.) ou organique (urée, etc.) ou un acide inorganique (acide nitrique, acide sulfurique, etc.) ou organique (acide formique, acide acétique, etc.). La précipitation doit avoir lieu à pH compris entre 4 et 13 comme il est connu de l'homme du métier, plus préférentiellement entre pH 5 et 9. Le co-précipité est filtré et lavé soigneusement en fonction de la nature des précurseurs et des agents de façon à limiter les teneurs en ions alcalins (sodium, potassium, etc.) à moins de 0,5 % et préférentiellement à moins de 0,1 % de masse par rapport aux oxydes. De même, les teneurs en anions (chlorure, sulfate, etc.) doivent être limitées à moins de 1 %, de préférence à moins de 0,3 % en masse. Le précipité obtenu peut être atomisé, puis mis en forme par extrusion, par pastillage ou par remise en suspension dans un solvant approprié pour former des billes. Dans tous les cas, les objets mis en forme sont séchés entre 40 et 150 °C, de manière préférée entre 70 et 120 °C, puis calcinés à des températures comprises entre 300 et 1100 °C, de manière préférée entre 350 et 800 °C.

Quelle que soit la méthode de préparation retenue, il est préférable d'avoir une teneur en dioxyde de titane d'au moins 10 % en masse, de préférence 23 % en masse et de manière encore plus préférée 50 % en masse. Dans la mesure du possible, le dioxyde de titane doit se trouver sous forme principalement amorphe ou micro-cristallisée, remarquable en cela par l'absence sur le diagramme de diffraction X de raies relatives aux formes cristallisées du dioxyde de titane connues par l'homme du métier.

Concernant la texture du catalyseur, il est important de maintenir la surface spécifique mesurée par la méthode BET connue par l'homme du métier, et le volume poreux à des valeurs correctes. Ainsi, le catalyseur aura en général une surface spécifique de 10 à 500 $m^2/g$, de préférence de 30 à 400 $m^2/g$ et de manière plus préférée de 40 à 300 $m^2/g$. De même, le volume poreux sera compris entre 0,1 $cm^3/g$ et 1,2 $cm^3/g$, et de manière préférée comprise supérieur à 0,2 $cm^3/g$. Enfin, la répartition poreuse sera de 0,001 à 0,1 micromètre.

Si l'on effectue la transestérification en l'absence de catalyseur, soit en autoclave, soit en lit fixe avec des supports inertes, comme le carbure de silicium, on peut obtenir, à certaines températures généralement supérieures ou égales à 250 °C, des conversions qui dépassent 80 %, mais à des WH très faibles et avec des temps de séjour très longs. La réaction thermique existe donc et il est parfois difficile de trancher entre l'effet catalytique et l'effet thermique, ce qui explique qu'avec des alumines simples, l'on peut obtenir des conversions élevées. Toutefois, le but du procédé de l'invention est d'obtenir ces conversions avec des temps de séjour raisonnables, donc à des VVH raisonnables.

Les conditions opératoires mises en oeuvre dépendent nettement du procédé choisi. Si l'on a recours à une réaction en discontinu, on peut travailler en une ou deux étapes, c'est à dire réaliser une première réaction jusqu'à 85 % à 95 % de conversion, refroidir en évaporant l'excès de monoalcool (par exemple méthanol), décanter la glycérine et finir la réaction en réchauffant à nouveau et en ajoutant de l'alcool pour obtenir une conversion totale. On peut aussi viser une conversion de 98 % en travaillant suffisamment longtemps en une seule étape.

Si l'on entreprend une réaction en continu, on peut travailler avec plusieurs autoclaves et décanteurs. Dans le premier, on réalise une conversion par exemple de 85 %, puis on décante en évaporant l'alcool et en refroidissant ; dans un deuxième réacteur, on achève la réaction de transestérification en rajoutant une partie de l'alcool que l'on a évaporé précédemment. On évapore finalement dans un évaporateur l'excès d'alcool et l'on sépare la glycérine et les esters par décantation.

Si l'on choisit un procédé continu en lit fixe, on peut avec avantage travailler à des températures de 150 à 250 °C, de préférence 170 à 210 °C, à des pressions de 30 à 70 bar, si l'on fabrique des esters méthyliques, la WH étant de préférence de 0,1 à 3, de préférence de 0,3 à 2, dans la première étape et le rapport massique alcool/huile variant de 3/1 à 0,1/1.

L'introduction de l'alcool peut être avantageusement fractionnée. L'introduction à deux niveaux dans le réacteur tubulaire peut s'opérer de la façon suivante : alimentation du réacteur avec l'huile et environ les 2/3 de l'alcool à mettre en jeu, puis introduction du complément d'alcool approximativement au niveau du tiers supérieur du lit catalytique.

Si l'on ne dépasse pas 220 °C, on obtient généralement un ester de même couleur que l'huile de départ et une glycérine incolore après décantation. L'ester peut être passé sur une résine, une terre et/ou du charbon actif, de même que la glycérine.

L'analyse des composés produits se fait, soit par chromatographie en phase gazeuse pour les esters et la glycérine, soit, plus rapidement, par chromatographie liquide par exclusion pour les esters. On constate que le procédé de l'invention, à l'inverse des procédés connus réalisés en catalyse basique homogène avec des monoalcools, ne produit que très peu d'esters de stérols. Les esters de stérols, qui sont des produits lourds, peuvent provoquer des dépôts dans les injecteurs.

Les exemples présentés ci-après ne limitent pas l'invention et ne servent qu'à l'illustrer.

## Synthèse de catalyseurs

### Catalyseur 1

Il consiste en un support alumine préformé sous forme de billes de 1,4 mm de diamètre, de surface spécifique SBET = 189 $m^2/g$ et de volume poreux V = 0,6 $cm^3/g$ est utilisé.

### Catalyseur 2

Le Catalyseur 2 est préparé par imprégnation d'isopropoxyde de zinc et d'isopropoxyde de titane sur le Catalyseur 1. L'alumine est calcinée à 400 °C pendant 1 h puis, après retour à la température ambiante, 23 g d'isopropoxyde de zinc et 23 g d'isopropoxyde de titane sont mélangés à 5 ml d'heptane, puis versé lentement sur 85 g d'alumine. L'ensemble est agité pendant 24 h. Le solide obtenu est placé à l'air ambiant pendant 72 h, puis séché en étuve. Le catalyseur est calciné à 500 °C pendant 4 h. L'analyse par diffraction des rayons X montre la présence de phase cristalline, caractéristique de la présence d'alumine gamma. Aucune raie caractéristique des phases rutile ou anatase n'est détectée. La surface spécifique mesurée par la méthode BET est de 141 $m^2/g$. Les teneurs en zinc et en titane mesurées par fluorescence X sont respectivement de 8,4 et 4,0 % en masse.

### Catalyseur 3

Le Catalyseur 3 est préparé par co-malaxage. On mélange 30 g de boehmite (Pural SB3) avec 35 g de gel de titane $TiO_2$ et 35 g d'oxyde de zinc ZnO en présence de 3 g d'acide nitrique à 70 % et 45 g d'eau. Les composants sont malaxés pendant 1 h pour former une pâte. La pâte ainsi obtenue est convertie en extrudés de 1,6 mm de diamètre qui sont séchés à 150 °C pendant 20 h et calcinés sous air à 600 °C pendant 3 h.

La surface spécifique mesurée par la méthode BET est de 62 $m^2/g$. La teneur en aluminium, en zinc et en titane mesurée par fluorescence X est respectivement de 14%, 24% et 17% en masse.

**Catalyseur 4**

Le Catalyseur 4 est préparé par imprégnation de pentyloxyde de bismuth et d'isopropoxyde de titane sur le Catalyseur 1. L'alumine est calcinée à 400 °C pendant 1 h, puis, après retour à la température ambiante, 15 g de pentyloxyde de bismuth et 27 g d'isopropoxyde de titane sont mélangés à 5 ml d'heptane, puis versé lentement sur 80 g d'alumine. L'ensemble est agité pendant 24 h. Le solide obtenu est placé à l'air ambiant pendant 72 h, puis séché en étuve. Le catalyseur est calciné à 500 °C pendant 4 h. L'analyse par diffraction des rayons X montre la présence de phase cristalline caractéristique de la présence d'alumine gamma. Aucune raie caractéristique des phases rutile ou anatase n'est détectée. La surface spécifique mesurée par la méthode BET est de 148 $m^2/g$. Les teneurs en bismuth et en titane, mesurées par fluorescence X, sont respectivement de 13,5 et 4,6 % en masse.

**Catalyseur 5**

Le Catalyseur 3 est préparé par co-malaxage. On mélange 30 g de boehmite (Pural SB3) avec 18 g de gel de titane $TiO_2$ et 51 g d'oxyde de bismuth $Bi_2O_3$ en présence de 3 g d'acide nitrique à 70 % et 45 g d'eau. Les composants sont malaxés pendant 1 h pour former une pâte. La pâte ainsi obtenue est convertie en extrudés de 1,6 mm de diamètre qui sont séchés à 150 °C pendant 20 h et calcinés sous air à 600 °C pendant 3 h.
La surface spécifique mesurée par la méthode BET sur le catalyseur obtenu est de 54 $m^2/g$. Les teneurs en aluminium, en bismuth et en titane, mesurées par fluorescence X, sont respectivement de 13%, 23% et 3,7% en masse.

**Exemple 1 (comparatif) :** Réaction en l'absence de catalyseur.

Dans un réacteur autoclave de 100 ml équipé d'un système d'agitation et d'un contrôle de température et de pression, on introduit 25 g d'huile de colza dont la composition est détaillée dans le tableau suivant et 25 g de méthanol.

| Glycéride d'acides gras | Nature de la chaîne grasse | % en masse |
|---|---|---|
| Palmitique | C16:0 | 5 |
| Palmitoléique | C16:1 | < 0,5 |
| Stéarique | C18:0 | 2 |
| Oléique | C18:1 | 59 |
| Linoléique | C18:2 | 21 |
| Linoléique | C18:3 | 9 |
| Arachidique | C20:0 | < 0,5 |
| Gadoléique | C20:1 | 1 |
| Béhénique | C22:0 | < 0,5 |
| Erucique | C22:1 | < 1 |

Le milieu est porté à 200 °C sous agitation. La pression atteint 32 bar.
Des échantillons sont prélevés après 2 heures, 5 heures et 7 heures. Sur chaque échantillon, après évaporation du méthanol en excès puis élimination du glycérol formé par décantation, la concentration en esters méthyliques est déterminée par chromatographie d'exclusion stérique. Elle est respectivement de 18%, 36% et 52%.

**Exemple 2 :** Réaction en présence de Catalyseur 3

Dans un réacteur autoclave de 100 ml équipé d'un système d'agitation et d'un contrôle de température et de pression, on introduit 25 g d'huile de colza dont la composition est détaillée dans l'Exemple 1, 25 g de méthanol et 1 g de Catalyseur 3. le milieu est porté à 200 °C sous agitation. La pression atteint 32 bar.
Des échantillons sont prélevés dans la phase liquide après 2 heures, 5 heures et 7 heures de réaction. Sur chaque échantillon, après filtration et évaporation du méthanol en excès puis élimination du glycérol formé par décantation, la concentration en esters méthyliques est déterminée par chromatographie d'exclusion stérique. Elle est respectivement de 66 %, 77 % et 87%.
Les concentrations de zinc, de titane et d'aluminium dans l'ester méthylique obtenu sont inférieures à 1 ppm. Les concentrations de zinc, de titane et d'aluminium dans le glycérol obtenu sont inférieures à 1 ppm. Ces résultats

confirment le caractère hétérogène de la catalyse.

Ceci permet l'utilisation du catalyseur dans un procédé de préparation d'esters carburants sans avoir à procéder à un traitement additionnel de purification de l'ester méthylique pour éliminer les traces de catalyseur résiduel.

Dans les mêmes conditions, le même catalyseur recyclé conduit à une concentration en esters méthyliques de 87 % après 7 heures de réaction, ce qui indique que le catalyseur n'est en rien dégradé et qu'il a conservé toute son activité. Cette opération a été répété encore deux fois et a conduit aux mêmes conclusions.

**Exemple 3**

On répète l'Exemple 2 en utilisant cette fois 150 g d'huile de colza, 150 g de méthanol et 25 g du Catalyseur 3.

La réaction est conduite à 200°C pendant 8 heures. Après filtration puis évaporation du méthanol en excès, suivie de l'élimination du glycérol formé par décantation, la concentration en esters méthyliques, déterminée par chromatographie d'exclusion stérique, est de 94 %.

Les concentrations de zinc, de titane et d'aluminium dans l'ester méthylique obtenu sont inférieure à 1 ppm. Les concentrations de zinc, de titane et d'aluminium dans le glycérol obtenu sont inférieure à 1 ppm. Ces résultats confirment le caractère hétérogène de la catalyse.

Dans les mêmes conditions, le même catalyseur recyclé conduit à une concentration en esters méthyliques de 93,5 % après 7 heures de réaction, ce qui indique que le catalyseur n'est en rien dégradé et qu'il a conservé toute son activité.

**Exemple 4 :** Réaction en présence de Catalyseur 5

On répète l'Exemple 2 en utilisant 1 g de Catalyseur 5.

Des échantillons sont prélevés dans la phase liquide après 2 heures, 5 heures et 7 heures de réaction. Sur chaque échantillon, après filtration et évaporation du méthanol en excès puis élimination du glycérol formé par décantation, la concentration en esters méthyliques est déterminée par chromatographie d'exclusion stérique. Elle est respectivement de 66 %, 78 % et 88 %.

Les concentrations de bismuth, de titane et d'aluminium dans l'ester méthylique obtenu sont inférieures à 1 ppm. Les concentrations de bismuth, de titane et d'aluminium dans le glycérol obtenu sont inférieures à 1 ppm. Ces résultats confirment le caractère hétérogène de la catalyse.

Ceci permet l'utilisation du catalyseur dans un procédé de préparation d'ester carburant sans avoir à procéder à un traitement additionnel de purification de l'ester méthylique pour éliminer les traces de catalyseur résiduel.

Dans les mêmes conditions, le même catalyseur recyclé conduit à une concentration en esters méthyliques de 88 % après 7 heures de réaction, ce qui indique que le catalyseur n'est en rien dégradé et qu'il a conservé toute son activité. Cette opération a été répétée encore deux fois et a conduit aux mêmes conclusions.

**Exemple 5**

On répète l'Exemple 4 en utilisant cette fois 150 g d'huile de colza, 150 g de méthanol et 25 g du Catalyseur 5.

La réaction est conduite à 200°C pendant 8 heures. Après filtration puis évaporation du méthanol en excès, suivie de l'élimination du glycérol formé par décantation, la concentration en esters méthyliques, déterminée par chromatographie d'exclusion stérique, est de 94 %.

Les concentrations de bismuth, de titane et d'aluminium dans l'ester méthylique obtenu sont inférieure à 1 ppm. Les concentrations de bismuth, de titane et d'aluminium dans le glycérol obtenu sont inférieure à 1 ppm. Ces résultats confirment le caractère hétérogène de la catalyse.

Dans les mêmes conditions, le même catalyseur recyclé conduit à une concentration en esters méthyliques de 93 % après 7 heures de réaction, ce qui indique que le catalyseur n'est en rien dégradé et qu'il a conservé toute son activité.

**Exemples 6 à 8**

On réalise la méthanolyse dans un appareil comportant un réacteur à lit fixe c'est à dire une colonne remplie, de diamètre égal à 1,9 cm et de longueur égale à 120 cm, chauffée par 3 coquilles qui entourent la colonne. Le préchauffage de l'huile et du méthanol se fait dans la colonne sur 10 $cm^3$ de billes de verre et la réaction sur 70 $cm^3$ de volume de Catalyseur 3. À la sortie de la colonne on a rajouté 20 $cm^3$ de carbure de tungstène et 5 $cm^3$ de billes de verre. Le dispositif en U renversé est constitué d'un réacteur tubulaire, d'un refroidissement sur la partie horizontale et d'un décanteur, qui constitue la deuxième branche. Sur la partie supérieure du décanteur, un système de purge gazeuse permet de réguler la pression, c'est à dire de maintenir celle-ci au départ avec de l'azote à la pression désirée

de 15 à 60 bar. Le décanteur possède à sa sortie inférieure une purge liquide. Lorsque le décanteur est à moitié plein, une vanne automatique s'ouvre pour vider partiellement le produit obtenu. Deux pompes injectent aux débits choisis et à pression constante l'alcool et l'huile dans la colonne et de bas en haut.

Les produits de réaction sont recueillis après 24 heures de passage à la WH désirée (WH = volume d'huile/ volume de catalyseur/ heure).

Après avoir soutiré le produit constitué de méthanol, de glycérol et d'ester, généralement présents en une seule phase, on évapore le méthanol, puis on sépare l'ester et le glycérol par décantation.

L'analyse de l'ester se fait par chromatographie d'exclusion stérique. Les résultats sont donc ceux obtenus sans aucune purification finale, si ce n'est celle qui consiste à évaporer l'excès de méthanol et à séparer l'ester de la glycérine par décantation, de préférence vers 50 °C.

Le tableau suivant présente les résultats obtenus après 24 heures de réaction.

La VVH est le volume d'huile injecté par volume de catalyseur et par heure. Le rapport R est le rapport en volume huile/alcool, noté H/A. La pression est la pression qui règne dans le décanteur, exprimée en bar.

La composition du mélange est exprimée en % masse.

Le temps de contact tient compte de la présence de méthanol ; il est déterminé par la relation :

$$\text{temps de contact} = \frac{70\ \text{cm}^3\ \text{de catalyseur x } 60(*)}{\text{volume en cm}^3\ \text{de l'huile + alcool injecté en 1 h}}$$

(*) 60 = temps en minutes.

Dans le tableau :

- E = esters (contient également les stérols)
- MG = monoglycérides
- DG = diglycérides qui ne contiennent pas d'esters de stérols, car il ne s'en forme pas dans ces conditions
- TG = triglycérides

| Méthanolyse de l'huile de colza avec le Catalyseur 3 | | | | | | | | | |
|---|---|---|---|---|---|---|---|---|---|
| Exemple | T (°C) | VVH | rapport H/A vol/vol | P (bar) | TG (%) | DG (%) | MG (%) | E (%) | t de contact (min) |
| 6 | 200 | 0,5 | 1 | 50 | 0,9 | 2,1 | 3,3 | 93,7 | 60 |
| 7 | 200 | 0,5 | 1,5 | 50 | 4,3 | 2,7 | 4,1 | 88,9 | 72 |
| 8 | 180 | 0,5 | 1 | 50 | 3,3 | 5,1 | 7,5 | 84,1 | 60 |

Des analyses de zinc et de titane par fluorescence X ont été effectuées sur les esters méthyliques et le glycérol obtenus. L'absence de zinc et de titane dans ces produits confirme le caractère hétérogène de la catalyse.

## Exemple 9

On répète la procédure des Exemples 6 à 8 en remplaçant l'huile de colza utilisé comme charge par un mélange d'esters dont la composition est identique à celle obtenue dans l'Exemple 6.

La composition de la phase ester obtenue est :

- Esters méthyliques : 99,3 %
- Monoglycérides : 0,6 %
- Diglycérides : 0,1 %
- Triglycérides : non détectés

Cette composition est compatible avec les spécifications requises pour un ester carburant pour moteur diesel.

## Exemples 10 à 12

On répète les Exemples 6 à 8 en utilisant le catalyseur de l'Exemple 5.

Les conditions sont indiquées dans le tableau suivant.

| Méthanolyse de l'huile de colza avec le Catalyseur 5 | | | | | | | | | |
|---|---|---|---|---|---|---|---|---|---|
| Exemple | T (°C) | VVH | rapport H/A vol/vol | P (bar) | TG (%) | DG (%) | MG (%) | E (%) | t de contact (min) |
| 10 | 200 | 0,5 | 1 | 50 | 0,8 | 1,7 | 3,0 | 94,5 | 60 |
| 11 | 200 | 0,5 | 1,5 | 50 | 3,6 | 2,8 | 3,6 | 90,0 | 72 |
| 12 | 180 | 0,5 | 1 | 50 | 2,9 | 5,1 | 7,1 | 84,9 | 60 |

Des analyses de bismuth et de titane par fluorescence X ont été effectuées sur les esters méthyliques et le glycérol obtenus. L'absence de bismuth et de titane dans ces produits confirme le caractère hétérogène de la catalyse.

**Exemple 13**

On répète la procédure des Exemples 10 à 12 en remplaçant l'huile de colza utilisé comme charge par un mélange d'esters dont la composition est identique à celle obtenue dans l'Exemple 10.

La composition de la phase ester obtenue est :

- Esters méthyliques : 99,5 %
- Monoglycérides : 0,4 %
- Diglycérides : 0,1 %
- Triglycérides : non détectés

Cette composition est compatible avec les spécifications requises pour un ester carburant pour moteur diesel.

**Revendications**

1. Procédé de fabrication d'au moins un ester d'acide gras et de glycérine à un haut degré de pureté, **caractérisé en ce qu'**il comprend la réaction d'une huile végétale ou animale avec un monoalcool aliphatique renfermant de 1 à 18 atomes de carbone, en présence d'au moins un catalyseur choisi parmi :

   - les combinaisons d'oxydes de zinc et de titane,
   - les combinaisons d'oxyde de zinc, d'oxyde de titane et d'alumine,
   - les combinaisons d'oxydes de bismuth et de titane et
   - les combinaisons d'oxyde de bismuth, d'oxyde de titane et d'alumine.

2. Procédé selon la revendication 1 **caractérisé en ce que** ledit monoalcool aliphatique renferme de 1 à 12 atomes de carbone.

3. Procédé selon la revendication 1 **caractérisé en ce que** ledit monoalcool aliphatique renferme de 1 à 5 atomes de carbone.

4. Procédé selon l'une des revendications 1 à 3 **caractérisé en ce que** l'on opère à une température entre 150 °C et 250 °C, sous une pression inférieure à 100 bar et avec un excès de monoalcool par rapport à la stoechiométrie huile/alcool.

5. Procédé selon la revendication 1 à 4 **caractérisé en ce que** ledit catalyseur est sous forme de poudre, d'extrudés ou de billes.

6. Procédé selon l'une des revendications 1 à 5 **caractérisé en ce que** le catalyseur présente une surface de 10 à 500 $m^2/g$, un volume poreux de 0,1 à 1,2 $cm^3/g$ et une répartition poreuse de 0,01 à 0,1 micromètre.

7. Procédé selon la revendication 6 **caractérisé en ce que** le catalyseur présente une surface de 30 à 400 $m^2/g$ et un volume poreux supérieur à 0,2 $cm^3/g$.

**8.** Procédé selon l'une des revendications 1 à 7 **caractérisé en ce que** la réaction est mise en oeuvre en discontinu.

**9.** Procédé selon l'une des revendications 1 à 8 **caractérisé en ce que** la réaction est mise en oeuvre en continu, soit en lit fixe, soit avec des autoclaves et décanteurs en série.

**10.** Procédé selon la revendication 9 **caractérisé en ce que** la réaction est mise en oeuvre en lit fixe, à une VVH de 0,1 à 3, de préférence 0,3 à 2.

**11.** Procédé selon l'une des revendications 1 à 10 **caractérisé en ce que** l'on met en oeuvre successivement :

- une transestérification initiale avec une conversion de l'huile en ester d'au moins 80 - 85 % ;
- l'évaporation du monoalcool en excès ;
- la décantation de la glycérine et de l'ester, ledit ester étant recyclé dans une deuxième étape pour subir une transestérification avec une partie du monoalcool récupéré dans la première évaporation ;
- puis une nouvelle évaporation du monoalcool, la décantation à froid et la séparation de la glycérine et de l'ester.

**12.** Procédé selon l'une des revendications 1 à 11 **caractérisé en ce que** l'huile de départ est une huile acide et on effectue une opération préalable de glycérolyse de l'acide gras libre, avec un catalyseur tel que celui utilisé pour la transestérification, à une température entre 180 et 220 °C et à une pression égale ou inférieure à 1 bar.

**13.** Procédé selon l'une des revendications 1 à 12 **caractérisé en ce que** l'on purifie l'ester obtenu, par passage sur une résine, une terre et/ou du charbon actif.

**14.** Procédé selon l'une des revendications 1 à 12, **caractérisé en ce que** l'on purifie l'ester obtenu, soit par distillation, soit par un lavage avec de la glycérine méthanolique pour en réduire la teneur en monoglycéride.

**15.** Procédé selon l'une des revendications 11 à 14 **caractérisé en ce que** la glycérine obtenue après évaporation de l'alcool est purifiée de façon définitive par passage sur une résine, une terre et/ou du charbon actif.

**16.** Procédé selon l'une des revendications 1 à 15 **caractérisé en ce que**, pour fabriquer l'ester éthylique, on utilise l'alcool éthylique en mélange avec une proportion de 1 à 50 % de méthanol.

**17.** Procédé selon l'une des revendications 1 à 16 **caractérisé en ce que** le catalyseur répond à la formule :

$$[(ZnO)_a - (TiO_2)_b]y\ [Al_2O_3]1\text{-}y,$$

cette formule pouvant également prendre la forme

$$[Zn_aTi_bO_{(a+2b)}]y\ [Al_2O_3]1\text{-}y,$$

a ayant une valeur de 0,5 à 5, b ayant une valeur de 0,5 à 5 et y ayant une valeur de 0,005 à 1.

**18.** Procédé selon la revendication 17 **caractérisé en ce que**, dans la formule :

$$[(ZnO)_a - (TiO_2)_b]y\ [Al_2O_3]_{1-y}$$

ou dans la formule :

$$[Zn_aTi_bO_{(a+2b)}]y\ [Al_2O_3]1\text{-}y,$$

y a une valeur de 0,005 à 0,995.

**19.** Procédé selon l'une des revendications 1 à 16 **caractérisé en ce que** le catalyseur répond à la formule :

$$[(Bi_2O_3)_a - (TiO_2)_b]y\ [Al_2O_3]1\text{-}y,$$

a ayant une valeur de 0,5 à 5, b ayant une valeur de 0,5 à 5 et y ayant une valeur de 0,005 à 1.

**20.** Procédé selon la revendication 19 **caractérisé en ce que**, dans la formule :

$$[(Bi_2O_3)_a - (TiO_2)_b]y\ [Al_2O_3]1\text{-}y,$$

y a une valeur de 0,005 à 0,995.

Office européen des brevets

# RAPPORT DE RECHERCHE EUROPEENNE

Numéro de la demande
EP 05 29 0798

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 924 185 A (INST FRANCAIS DU PETROL) 23 juin 1999 (1999-06-23)<br>* page 4, alinéa 22 *<br>* exemples *<br>----- | 1-18 | C11C3/10<br>C07C67/03<br>C07C69/24<br>C07C69/52 |
| D,A | US 5 908 946 A (ROUXEL JEAN-JACQUES ET AL) 1 juin 1999 (1999-06-01)<br>* le document en entier *<br>----- | 1 | |
| A | PETERSON G R ET AL: "Rapeseed oil transesterification by heterogenous catalysis"<br>JOURNAL OF THE AMERICAN OIL CHEMISTS' SOCIETY, AMERICAN OIL CHEMISTS' SOCIETY. CHAMPAIGN, US,<br>vol. 61, no. 10, octobre 1984 (1984-10), pages 1593-1597, XP002170978<br>ISSN: 0003-021X<br>* le document en entier *<br>----- | 1 | |

| DOMAINES TECHNIQUES RECHERCHES (Int.Cl.7) |
|---|
| C07C<br>B01J<br>C11C |

Le présent rapport a été établi pour toutes les revendications

| Lieu de la recherche | Date d'achèvement de la recherche | Examinateur |
|---|---|---|
| La Haye | 1 septembre 2005 | Kardinal, S |

2

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 29 0798

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-09-2005

| Document brevet cité au rapport de recherche | Date de publication | Membre(s) de la famille de brevet(s) | Date de publication |
|---|---|---|---|
| EP 0924185 A | 23-06-1999 | FR 2772756 A1 | 25-06-1999 |
| | | AT 224352 T | 15-10-2002 |
| | | DE 69808029 D1 | 24-10-2002 |
| | | DE 69808029 T2 | 06-02-2003 |
| | | EP 0924185 A1 | 23-06-1999 |
| | | ES 2184211 T3 | 01-04-2003 |
| | | US 6147196 A | 14-11-2000 |
| US 5908946 A | 01-06-1999 | FR 2752242 A1 | 13-02-1998 |
| | | IT 1293520 B1 | 01-03-1999 |

EPO FORM P0460

Pour tout renseignement concernant cette annexe : voir Journal Officiel de l'Office européen des brevets, No.12/82